# EUROPEAN PATENT SPECIFICATION

(11) **EP 3 124 048 B1**
(45) Date of publication and mention of the grant of the patent: **13.05.2020**
(21) Application number: 16181422.3
(22) Date of filing: 27.07.2016
(51) Int. Cl.: A61K 45/06, A61K 31/704, A61K 31/734, A61K 36/185, A61K 36/484, A61P 1/04, A61K 33/00, A61K 33/10

(54) **COMPOSITION FOR ORAL USE IN THE TREATMENT OF GASTRO-OESOPHAGEAL REFLUX DISEASE OR DISCOMFORT**
ORALE ZUSAMMENSETZUNG ZUR BEHANDLUNG VON GASTROÖSOPHAGEALEN KRANKHEITEN ODER LEIDEN
COMPOSÉE ORALE DESTINÉE AU TRAITEMENT DES MALADIES OU AFFECTIONS GASTRO-OESOPHAGIENNES

(30) Priority: 30.07.2015 IT UB20152623
(43) Date of publication of application: 01.02.2017
(73) Proprietor: Neilos S.r.l., 80063 Piano di Sorrento (NA) (IT)
(72) Inventor: DI MAIO, Umberto, 80063 Piano di Sorrento (NA) (IT)
(74) Representative: Hoffmann Eitle

(56) References cited:
- WO-A1-2010/092468
- WO-A1-2015/105346
- WO-A1-2016/048059
- US-A1- 2010 143 319
- US-A1- 2013 210 762
- US-A1- 2015 037 315
- MANDEL K G ET AL: "Review article: Alginate-raft formulations in the treatment of heartburn and acid reflux", ALIMENTARY PHARMACOLOGY & THERAPEUTICS, BLACKWELL SCIENTIFIC PUBLICATIONS LTD., CAMBRIDGE, GB, vol. 14, no. 6, 1 June 2000 (2000-06-01), pages 669-690, XP002402797, ISSN: 0269-2813
- BROWN RACHEL ET AL: "Effect of GutsyGum(tm), A Novel Gum, on Subjective Ratings of Gastro Esophageal Reflux Following A Refluxogenic Meal", JOURNAL OF DIETARY SUPPLEMENTS, INFORMA HEALTHCARE, NEW YORK, vol. 12, no. 2, 1 June 2015 (2015-06-01), pages 138-145, XP008179288, ISSN: 1939-0211, DOI: 10.3109/19390211.2014.950783 [retrieved on 2014-08-21]

## Description

The present invention relates to a composition for use in the treatment of gastroesophageal reflux diseases or disorders, preferably in the symptomatic treatment of gastroesophageal reflux diseases or disorders. Said composition is for oral use and, from a physical standpoint, is in any pharmaceutically acceptable form of administration. Furthermore, said composition comprises a mixture comprising sodium alginate, calcium carbonate, sodium bicarbonate, the digestive enzyme actinidin from *Actinidia chinensis Planch* (Kiwi) dry fruit extract, having a content of actinidin of 16000 CDU/g and liquorice *(Glycyrrhiza glaba L.)* in the form of a dry root extract containing 3% glycyrrhizic acid.

It is known that today an increasing number of people suffer from gastroesophageal reflux disease or disorders that require temporary or prolonged treatments, according to severity, with specific compositions or drugs. Gastroesophageal reflux disease (GERD) is a clinical condition characterised by the reflux of gastroduodenal content into the esophagus, with the appearance of symptoms able to interfere with the quality of life. It is estimated that retrosternal pyrosis occurs at least once a day in 4-7% of the general population and in 34-44% at least once a month. The incidence of GERD is estimated to be 4.5/100.000, with a marked increase over 40 years of age.

Gastroesophageal reflux refers to an "involuntary and unconscious passage of part of the gastric contents into the esophagus, without the joint participation of gastric and abdominal muscles". The esophagus is a canal, 25-30 cm long, which connects the mouth with the stomach, and along the course of which it is possible to identify two sphincter structures: the first sphincter structure is between the hypopharynx and the cervical tract of the esophagus (UES, upper esophageal sphincter), whilst the second sphincter structure, called the lower esophageal sphincter (LES), is at the level of the gastro-esophageal junction. The latter is a high pressure zone that represents the main anti-reflux structure, thanks to its location between the negative-pressure intrathoracic zone and the positive-pressure intra-abdominal zone. Therefore, under normal conditions, an increase in abdominal pressure has repercussions at the level of the LES, preventing the return of ingested material into the esophagus. Under physiological conditions the LES is closed and relaxes for a time of about 3-10 seconds following deglutition.

Other anatomical structures, in addition to the LES, which contribute to maintaining the anti-reflux barrier are:
- The angle of His, an acute angle formed between the esophagus and the gastric fundus;
- The phrenoesophageal ligament;
- The diaphragmatic collar, consisting of fasciae of the diaphragm, which wrap around the esophagus like a scarf and constrict the lumen during inspiration.

There are numerous factors underlying the pathogenesis of GERD, such as, for example:
1. Insufficiency of the anti-reflux barrier of the lower esophageal sphincter, which has the purpose of mechanically preventing gastric juices from flowing up into the esophagus.
2. Delay in gastric emptying due to anatomical anomalies or functional alterations: (i) anatomical anomalies: stenosis of the pylorus (it is the terminal region of the stomach, which regulates the passage of gastric contents in the duodenum); (ii) functional alterations: motor alterations of the fundus (region responsible for the emptying of liquids).
3. Insufficiency of the esophageal clearing mechanism, which has the purpose of minimising contact between the esophageal mucosa and gastric juices by acting both through esophageal peristalsis and the neutralisation of acidic residues thanks to saliva.
4. Gastric hyperacidity.
5. Aggressiveness of the gastric contents that flow back into the esophagus, due to the action of HCl.
6. Duodenogastric reflux with the passage of pancreatic and biliary secretions into the stomach, which in the case of gastroesophageal reflux, can cause more severe lesions. Other predisposing factors include smoking, improper dietary behaviour (abundant meals, high-fat foods, caffeine), drugs, pregnancy and obesity, which can esacerbate GERD - gastroesophageal reflux disease. A hiatal hernia (extension of a portion of the stomach into the thorax through an opening in the diaphragm called the esophageal hiatus) also frequently accompanies GERD and can contribute to the prolonged exposure to gastroduodenal contents. In general, the walls of the esophageal hiatus adhere closely to the esophagus, but it can happen that the anchorage structures of the lower portion of the esophagus lose tone, favouring the ascent of a small part of the stomach into the thorax.

In any event, whatever the cause may be, the frequent and repeated contact of regurgitated gastric materials with the esophageal mucosa exerts a harmful action on the latter, which is all the more serious the longer the contact time and the lower the pH of the reflux (regurgitated gastric materials). Over time, the persistent phlogistic action affecting the esophageal mucosa leads to an inflammatory reaction that can evolve into ulcerations, stenosis and so-called columnar metaplasia (or Barrett's epithelium, the single most important risk factor for the development of esophageal adenocarcinoma). Symptoms considered to be typical are retrosternal pyrosis (defined by the patient as a burning sensation that starts at the stomach or the lower portion of the thorax and rises toward the neck) and regurgitation (perception of acidic and bitter tasting liquid inside the oral cavity), symptoms whose specificity for GERD is equal to 89 and 95%, respectively. Frequent albeit less specific symptoms are odynophagia, dysphagia, belching, epigastric pain, bloating and digestive difficulties. Some of these symptoms characterise the diagnosis of functional dyspepsia and it is known that between 10% and 17% of patients requiring medical intervention for dyspepsia have GERD - gastroesophageal reflux disease.

WO 2015/105346 discloses pharmaceutical compositions for the treatment of gastric reflux comprising an sodium alginate, calcium carbonate and sodium bicarbonate and an extract of *Glycyrrhiza.* Flavourings, including kiwi extract, may also be added.

US 2013/210762 discloses pharmaceutical compositions for the treatment of dyspepsia comprising sodium alginate, sodium bicarbonate and calcium carbonate.

Alim. Pharmac. Ther. 2000, 14(6), 669 discloses alginate-based antacids such as Gaviscon® comprising sodium alginate, sodium bicarbonate and calcium carbonate.

US 2015/037315 discloses dietary supplements for the treatment of digestive dysfunction and gastrointestinal disorders (or their symptoms), such as gastric reflux, comprising extracts of kiwi fruit and amylase.

US 2010/143319 discloses that extracts from kiwi fruit are useful for managing gut health and treating gastrointestinal tract disorders, such as gastric reflux.

J. Diet. Suppl. 2015, 12(2), 138 discloses mixtures of calcium carbonate and liquorice extract for use in the treatment of gastrooesophageal reflux.

WO 2010/092468 discloses compositions comprising alginates, such as sodium alginate, and antacids, including calcium carbonate and sodium bicarbonate, for use in the treatment of gastrooesophageal reflux and dyspepsia.

Therefore, there is a constantly felt need to be able to have compositions or drugs for the treatment of gastroesophageal reflux that are well tolerated, effective and, if possible, free of side effects, especially in the case of individuals who have to take anti-reflux drugs or compositions for extended periods of time. After lengthy, intense research and development activity, the Applicant has identified a combination (mixture) of compounds and substances that are capable of acting effectively, in a mutually synergistic manner, against gastroesophageal reflux disease or disorders.

Advantageously, said combination (mixture) of compounds and substances is well tolerated and shows no side effects, even if administered over extended periods in the form of a composition for oral use.

The present invention relates to a composition comprising a mixture, having features as defined in the appended independent claim.

The present invention relates to said composition for use as a medication, having features as defined in the appended claims.

The present invention relates to said composition for oral use in the treatment of gastroesophageal reflux diseases or disorders, having features as defined in the appended claims. Preferably, the treatment of gastroesophageal reflux disease or disorders includes reducing gastric emptying times.

Preferred embodiments of the present invention are illustrated below.

The Applicant has developed a mixture of compounds and substances that perform a mutually synergistic activity.

According to the invention, the mixture comprises sodium alginate, calcium carbonate, sodium bicarbonate, the digestive enzyme actinidin from *Actinidia chinensis Planch* (Kiwi) dry fruit extract, having a content of actinidin of 16000 CDU/g, and liquorice *(Glycyrrhiza glaba L.)* in the form of a dry root extract containing 3% glycyrrhizic acid.

Alginates, such as sodium alginate, are natural polysaccharides which precipitate in contact with gastric acid, forming a low-density gel (with an almost neutral pH) in just a few minutes. The change in pH triggered by bicarbonates and carbonates, which are likewise present together in the mixture of the invention, free carbon dioxide, which is trapped within the alginate gel, causing it to float on the gastric contents. The alginate gel forms in the portion of stomach near the gastroesophageal junction, precisely where the acid pocket develops. In this manner, the reflux of acid from stomach to the esophageal canal is blocked or greatly reduced.

Sodium bicarbonate and calcium carbonate together are effective in neutralising HCI, the fundamental component of the gastric juice produced by the parietal cells of the stomach. Once they have arrived in the stomach, bicarbonates, such as sodium bicarbonate, react with the HCI, producing carbon dioxide, and ensure an immediate antacid action. The carbon dioxide thus produced is trapped within the alginate gel, causing it to float on the gastric contents.

The second cause, in order of importance, of the appearance of symptoms of gastroesophageal reflux, is delayed gastric emptying. The longer food remains in the stomach, the more likely it is that an episode of gastroesophageal will occur.

Digestive enzymes, taken together, are essential for digesting food proteins, which are reduced into molecular fragments that are small enough to enable them to be absorbed. Through their action, digestive enzymes are capable of breaking the peptide bonds that join together the various amino acids and whose repeated concatenation gives rise to protein molecules; this facilitates the process of digestion.

The use of the digestive enzyme actinidin from *Actinidia chinensis Planch* (kiwis) in the present mixture, in association with sodium alginate, sodium bicarbonate and calcium carbonate serves to modify gastric emptying times, reducing them. This effect is due to the combined, synergistic action due to the presence of the individual compounds/substances.

The effect of gastric emptying obtained in shorter times and with improved efficacy and control is due to a combined, synergistic action, which is obtained thanks to the presence of the individual compounds/substances, including the association between said digestive enzyme actinidin from *Actinidia Chinensis Planch* (kiwis) and liquorice.

Preferably, said composition for oral use in the treatment of gastroesophageal reflux disease or disorders includes reducing gastric emptying times, said composition comprising a mixture having features as defined in the appended claim.

Liquorice *(Glycyrrhiza glaba L.)* belongs to the family *Leguminosae* and contains, among its main constituents, triterpene saponins, most notably glycyrrhizin (glycyrrhizic acid). The liquorice *(Glycyrrhiza glaba L.)* present in the mixture in the form of a dry extract (d.e.) of the root exerts, in synergy with the other compounds or substances present in the mixture, an effective action in reducing gastric secretion, which is important for reducing, to the extent possible, the amount of acid produced.

Advantageously, the mixture of the present invention and, consequently, the composition containing it are capable of exerting (i) an anti-reflux action aimed at reducing the passage of acid from the stomach to the esophagus, (ii) an antacid action, aimed at neutralising gastric hyperacidity, (iii) a digestive action, aimed at reducing gastric emptying times and/or (iv) an anti-ulcer/anti-inflammatory action.

The composition of the present invention comprises said mixture, which comprises sodium alginate, sodium bicarbonate, calcium carbonate, said digestive enzyme actinidin from *Actinidia chinensis Planch* (kiwis) and liquorice *(Glycyrrhiza glaba L.)* in the form of a dry root extract; said composition being for use in the treatment of gastroesophageal reflux disease or disorders, preferably in the symptomatic treatment of gastroesophageal reflux disease or disorders. Said composition being for oral use in any pharmaceutically acceptable form of administration, such as, for example, in the form of capsules, pastilles, tablets, soluble or water-dispersible granules or powders packaged in sticks or sachets, syrup or solution. Preferably, said composition for use of the present invention can also be validly applied as a gastroprotective agent in the treatment of gastroesophageal reflux disease or disorders that includes normalising and improving digestive function.

Along with an anti-reflux, antacid and anti-inflammatory action, the composition for use of the present invention associates a targeted action performed by the digestive enzyme actinidin from *Actinidia chinensis Planch* (kiwis), thus enabling an appreciable reduction in gastric emptying times, thanks to faster and more effective digestion, and a simultaneous reduction in the amount of hydrochloric acid produced which comes into contact with the gastric mucosa. The reduction in gastric emptying times, the reduction in the amount of hydrochloric acid produced and the reduction in the times of contact between the hydrochloric acid and the gastric mucosa together enable the composition for use of the present invention to improve the symptoms of GERD.

In a preferred embodiment of the present invention, the composition for use comprises a mixture containing, relative to 10 ml of volume: (i) Sodium alginate: 500 mg; (ii) Sodium bicarbonate: 150 mg; (iii) Calcium carbonate: 266.66 mg; (iv) the digestive enzyme actinidin from *Actinidia chinensis Planch* (kiwis) dry fruit extract, actinidin content 16000 CDU/g: 20 mg; and (v) *Glycyrrhiza glabra L.* (liquorice) dry root extract containing 3% glycyrrhizic acid: 30 mg. The composition is prepared using techniques and equipment known to the person skilled in the art, who is capable of mixing together the various components/substances and creating a pharmaceutical form, e.g. capsules, pastilles, tablets, soluble or water-dispersible granules or powders packaged in sticks or sachets, syrup or solution.

The composition for use of the present enables the synergy of the following actions:
- Anti-reflux action, aimed at reducing the passage of acid from the stomach to the esophagus;
- Antacid action, aimed at neutralising gastric hyperacidity;
- Digestive action, aimed at reducing gastric emptying times;
- Anti-ulcer/anti-inflammatory action.

As already noted, GERD is caused by the involuntary and unconscious passage of part of the gastric contents in the esophagus; this passage provokes damage to the esophageal mucosa which varies based on the number of refluxes over time and the amount of acid that comes into contact with the mucosa. The latter factor can be particularly influenced by gastric emptying times; indeed, the longer the food ingested through the diet remains in the stomach, the greater will be the amount of acid produced by parietal cells, with a consequent increase in the symptoms associated with GERD.

Once orally administered, the alginates contained in the product of the invention will form, as previously described, a "plug" which reduces the amount of gastric juice rising from the stomach towards the esophagus, thus minimising the time of exposure of the mucosa to acids. Simultaneously, the sodium bicarbonate and calcium carbonate react with the HCI, neutralising it; the antacid action reduces the amount of HCI that will spill into the esophagus upon the next reflux, thus reducing the damage to the esophageal mucosa.

The digestive enzyme actinidin from *Actinidia chinensis Planch* (kiwis) is capable of breaking the peptide bonds that join together the various amino acids and whose repeated concatenation gives rise to protein molecules; this facilitates the process of digestion. The ratio between gastric emptying times and the times of exposure of the esophagus to gastric juices is difficult to evaluate; a delay in gastric emptying times influences the amount of acid that gets past the gastroesophageal valve, but does not influence the total number of refluxes. Reducing gastric emptying times reduces the amount of acid produced over time; therefore, the action of the digestive enzymes is to reduce the amount of acid that passes through the gastroesophageal valve, thus reducing the times of exposure of the mucosa to the damaging action of the acids and further protecting the gastric mucosa from the prolonged action of the acids. Finally, the anti-inflammatory action of the liquorice improves the symptoms associated with prolonged exposure of the mucosa to the action of gastric juices.

### Experimental part

### Study conducted on the composition of the present invention for the treatment of gastroesophageal reflux.

An assessment was made of the effect of a composition of the present invention C1 and the individual constituents thereof (in order to observe a synergistic effect among the individual components) (i) on gastric emptying, (ii) on reflux esophagitis, (iii) on gastric secretion (volume of gastric contents, pH and total acidity) and (iv) on gastric ulcer.

### Material and methods: Animals

Male mice of the ICR strain supplied by the company Charles River and weighing 20-25 g were used. The animals were housed in temperature-controlled rooms (temperature of 23±2 °C, humidity 50±2%, 12-hour light-dark cycles).

The mice had free access to water and food, which consisted of a standard diet supplied by the company Mucedola Mangimi (Settimo Milanese, Italy).

All experiments were performed in observance of Legislative Decree no. 116 of 27 January 1992 and according to the guidelines of the Council of the European Union (86/609/EEC and 2010/63/EU).

### Gastric emptying

Gastric emptying was evaluated with the method described by Smits and Lefebvre (1996). For the purpose of determining the gastric emptying, the animals were orally administered a marker (0.2 ml/mouse of a suspension containing 50 mg of phenol red in 100 ml of 1.5% carboxymethylcellulose). After twenty minutes, the animals were sacrificed in a CO₂ saturated atmosphere and the stomach was removed. The stomach was subsequently positioned inside a test tube containing 4 ml of saline solution; after 20 seconds of shaking, 2 ml of NaOH 1 M was added to each test tube in order to achieve the maximum colour intensity.

Spectrophotometric analysis (560 nm) was carried out on 1 ml of this solution. The percentage of gastric emptying was calculated according to the following formula: 100X(1-[amount of phenol red present in the stomach after 20 min)/(amount of phenol red present in the stomach at time 0]).

### Reflux esophagitis and gastric secretion/ulcer

Reflux esophagitis and gastric secretion/ulcer were induced using the experimental model of pylorus ligation described by Shay and coll. (1945). The animals, fasted for 24 hours but with free access to water, were anaesthetised, the abdomen was opened and the pylorus was ligated. Four hours after the surgical procedure (time necessary to determine a submaximal lesion of the mucosa of the esophagus), the mice were sacrificed in a CO₂ saturated atmosphere and the esophagus and stomach were removed for evaluation of: 1) the macroscopic esophageal and gastric damage, 2) the degree of esophageal and gastric inflammation (activity of myeloperoxidase) and 3) the characteristic parameters of gastric secretion (volume of the gastric contents, pH and total acidity).

### 1) Macroscopic esophageal and gastric damage

The esophagus (opened longitudinally) and stomach (opened along the greater curvature), were spread out on a polystyrene support and analysed with the aid of a microscope so as to identify lesions of the mucosa. The damage to the mucosa was determined using a scoring scale that takes into consideration the severity and extent of hyperaemia and haemorrhagic erosions.

### 2) Degree of inflammation of the esophagus and stomach

The degree of inflammation of the esophagus and stomach was assessed by determining the activity of myeloperoxidase (MPO). MPO is a protein that is found in the azurophilic granules of polymorphonuclear neutrophils and is used as a marker of leukocyte infiltration. In order to assess the activity of MPO, the tissues were subjected to three homogenisation cycles lasting 10 sec each, at maximum speed, in a suitable lysis buffer defined as MOPS (0.5% HTAB in MOPS 10 Mm), in a ratio of 50 mg of tissue/lml of MOPS. The homogenates underwent centrifugation at 12,000 rpm for 20 min at 4°C. Subsequently, the supernatants were incubated with NaPP (sodium phosphate buffer pH 5.5) and TMB 16 mM (tetramethylbenzidine) and, after five minutes of incubation at room temperature, H₂O₂ diluted in NaPP was added. The reaction was blocked with cold acetic acid 2M and a reading of 1 ml of the reaction solution was taken on a spectrophotometer at a wavelength (X) of 650-655 nm. The values obtained were compared to a standard MPO curve and the results expressed as U/ml of MPO.

### 3) Evaluation of the characteristic parameters of gastric secretion

The gastric contents were collected for the purpose of determining the pH value and total quantity of gastric juice (volume). Subsequently, 2 ml of distilled water was added to the gastric contents, which were centrifuged at 5000 rpm for 15 min for the determination of total acidity, carried out on the supernatant by titration up to pH=7 (using 2% phenolphthalein as an indicator) with NaOH 0.01 N. The total acidity was expressed as mequiv.[H+]/ml/4 h.

### Pharmacological treatment

The mice were divided into 6 groups:
Group 1: control, composition C1 based on a standard diet.
Group 2: composition of the present invention C2 based on: sodium bicarbonate, sodium alginate, calcium carbonate, digestive enzymes and liquorice,
Group 3: composition C3 based on: sodium bicarbonate, sodium alginate and calcium carbonate,
Group 4: composition C4 based on: sodium bicarbonate, sodium alginate, calcium carbonate and digestive enzymes,
Group 5: composition C5 based on: [composition C2] + a mixture of plant extracts consisting of prickly pear d.e., olive d.e. and mint.
Group 6: composition C6 based on: composition C5 at half the dosage of C5.

The treatments were administered orally 30 minutes before the oral administration of the marker adopted to measure gastric emptying (gastric emptying assay) and immediately after ligation of the pylorus (gastric secretion assay).

The present invention relates to a composition for oral use comprising a mixture which comprises or, alternatively, consists of sodium bicarbonate, sodium alginate, calcium carbonate, digestive enzymes (as described above) and liquorice, together with a mixture of plant extracts comprising or, alternatively, consisting of prickly pear d.e, olive d.e. and mint.

A RefluWard™ embodiment comprises:
- vegetable glycerine,
- sodium alginate,
- calcium carbonate,
- sodium bicarbonate,
- mixture of plant extracts: *Opuntia ficus-indica Mill.* (prickly pear) dry cladode extract, *Olea europaea L.* (olive) dry leaf extract containing 3.7% polyphenols, *Glycyrrhiza glabra L.* (liquorice) dry root extract containing 3% glycyrrhizic acid, *Mentha x piperita L.* (mint) dry leaf extract 1:4 and *Actinidia chinensis Planch* (kiwi) dry fruit extract, actinidin content 16000 CDU/g,
- preservatives potassium sorbate and sodium benzoate,
- flavourings.

For two 10 ml stick packs for oral use:
Mixture of plant extracts 326 mg, of which:
- Prickly Pear d.e.: 104 mg,
- Olive d.e.: 76 mg,
- Polyphenols (derived from prickly pear d.e. and olive d.e.): 12 mg,
- Liquorice d.e.: 60 mg,
- Mint d.e. 1:4, 60 mg,
- Kiwi d.e. (actinidin 16000 CDU/g): 40 mg.

For 100 ml:
Mixture of plant extracts 1630 mg, of which:
- Prickly Pear d.e.: 520 mg,
- Olive d.e.: 380 mg,
- Polyphenols (derived from prickly pear d.e. and olive d.e.): 60 mg,
- Liquorice e.s: 300 mg,
- Mint d.e. 1:4, 300 mg,
- Kiwi d.e. (actinidin 16000 CDU/g): 200 mg.

*Opuntia ficus-indica (L.) Mill.* (prickly pear) is a plant belonging to the family *Cactaceae*; the cladodes of the Prickly Pear (polymers containing mucilage and pectin) perform a cicatrising activity useful for treating gastric ulcers.

*Olea europaea L.* (olive) is a plant belonging to the family Oleaceae. The olive has a high antioxidant power by virtue of the active substances present in the extracts thereof, including, among others, oleic acid, polyphenols and squalenes. The antioxidant action of the olive is effective in the treatment of gastroesophageal reflux and other correlated pathologies such as esophagitis. The reflux of gastric juices into the esophagus is a major factor predisposing to esophagitis; the latter can arise due not only to the contact between the acids contained in the stomach and the esophageal mucosa, but can also depend on other factors.

*Mentha x Piperita L.* (mint) is a plant belonging to the family *Lamiaceae,* useful in the symptomatic treatment of digestive disorders such as dyspepsia (for example, spastic disorders in the upper gastrointestinal tract) and gastritis (an inflammatory disease of the gastric mucosa). Both of these pathologies are correlated to GERD: the former represents a factor predisposing to the disease, whilst the latter can be a consequence of continuous contact of gastric juices with the mucosa of the stomach.

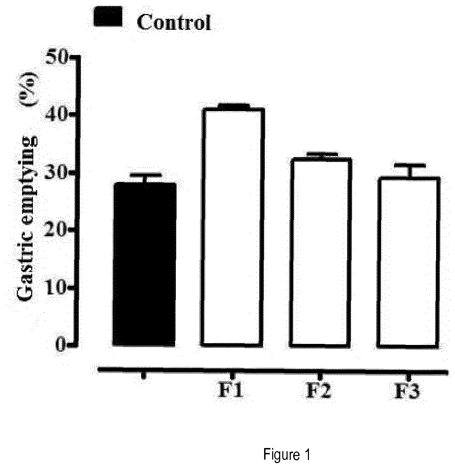

The Applicant conducted the following *in vivo* experimental test, in which it tested three formulations, F1 (composition of the present invention), F2 and F3 - see Table 1 (Composition F1), Table 2 (Composition F2), Table 3 (Composition F3) and Figure 1.

**Table 1**

| Group F1: Neilos | |
|---|---|
| INGREDIENTS | PER 10 mL |
| Sodium alginate | 500 mg |
| Calcium carbonate | 266.66 mg |
| Sodium bicarbonate | 150 mg |
| Glycyrrhiza glabra L. (liquorice) dry root extract containing 3% glycyrrhizic acid | 30 mg |
| Actinidia chinensis Planch. (kiwi) dry fruit extract, actinidin content 16000 CDU/g | 20 mg |
| Mint Flavouring | 5 mg |
| Vegetable Glycerine | q.s. to 10 mL |

**Table 2**

| Group F2 | |
|---|---|
| INGREDIENTS | PER 10 mL |
| Sodium alginate | 500 mg |
| Calcium carbonate | 266.66 mg |
| Sodium bicarbonate | 150 mg |
| Mint Flavouring | 5 mg |
| Potassium Sorbate, Sodium Benzoate | 20 mg |
| Vegetable Glycerine | q.s. to 10 mL |

**Table 3**

| Group F3 | |
|---|---|
| INGREDIENTS | PER 10 mL |
| Sodium alginate | 500 mg |
| Calcium carbonate | 266.66 mg |
| Sodium bicarbonate | 150 mg |
| Actinidia chinensis Planch. (kiwi) dry fruit extract, actinidin content 16000 CDU/g | 20 mg |
| Mint Flavouring | 5 mg |
| Potassium Sorbate, Sodium Benzoate | 20 mg |
| Vegetable Glycerine | q.s. to 10 mL |

### Materials and methods

### Animals

Male mice of the ICR strain supplied by the company Charles River and weighing 20-25 g were used. The animals were housed in temperature-controlled rooms (temperature of 23±2 °C, humidity 50±2%, 12-hour light-dark cycles) and had free access to water and food (consisting of a standard diet supplied by the company Mucedola Mangimi (Settimo Milanese, Italy) except for 20 minutes (or 18 hours) before the experiment on gastric emptying. All experiments were performed in observance of Legislative Decree no. 116 of 27 January 1992 and according to the guidelines of the Council of the European Union (86/609/EEC and 2010/63/EU).

### Gastric emptying

Gastric emptying was evaluated with the method described by Smits and Lefebvre (1996) [1]. The animals were fasted for 18 hours and subsequently allowed to feed 20 minutes before the start of the experiment with the aim of assuring that the gastric food contents in each animal would be as similar as possible. For the purpose of determining the gastric emptying, the animals were orally administered a marker (0.2 ml/mouse of a suspension containing 50 mg of phenol red in 100 ml of 1.5% carboxymethylcellulose). After twenty minutes, the animals were sacrificed in a CO₂ saturated atmosphere and the stomach was removed. The stomach was subsequently positioned inside a test tube containing 4 ml of saline solution; after 20 seconds of shaking, 2 ml of NaOH 1 M was added to each test tube in order to achieve the maximum colour intensity. Spectrophotometric analysis (560 nm) was carried out on 1 ml of this solution. The percentage of gastric emptying was calculated according to the following formula: 100 x (1-[amount of phenol red present in the stomach after 20 min)/(amount of phenol red present in the stomach at time 0]).

### Pharmacological treatment

The treatments were administered orally 30 minutes before the oral administration of the marker adopted to measure gastric emptying (gastric emptying assay). The formulations were suspended in glycerol in order to administer a final volume of 100 µl per mouse.

### Statistical analysis

The results were expressed as the mean ± standard error (SEM) of the *n*=8-10 experiments and analysed using Student's t-test to evaluate the individual treatments vs the mean of the control group and analysis of variance followed by the Tukey-Kramer test for the multiple comparison and analysis of the means of the treatments. A value of P<0.05 was considered statistically significant.

### Results

### Gastric emptying

Figure 1 shows the results with regard to the effect of the formulations F1, F2 and F3 on gastric emptying in animals fasted for 18 hours and subsequently allowed to feed for 20 minutes before the start of the experiment so as to enable the presence of a same quantity of food inside the stomach of all animals. As may be observed, formulation F1, orally administered at a dose of 10 µl/mouse, was able to significantly increase gastric emptying (maximum effect: Formulation F1 41.1±0.6). In contrast, formulations 2 and 3, administered at the same dose of 10 µl/mouse, proved inactive.

Figure 1 relates to the effect of the formulations F1-F2-F3 (10 µl/mouse, *per os)* on gastric emptying evaluated in mice fasted for 18 hours and subsequently allowed to feed for 20 minutes before the start of administration of the formulations. The formulations were administered 30 minutes before the start of the experiment. Each point represents the mean of 8-10 animals. *P<0.05 and ***P<0.001 *vs* the control; °°P<0.01.

Figure 1 shows the results with regard to the effect of the formulations F1-F2-F3 on gastric emptying in animals fasted for 18 hours and subsequently allowed to feed for 20 minutes before the start of the experiment so as to enable the presence of a same quantity of food inside the stomach of all animals.

As may be observed, formulation F1, orally administered at a dose of 10 µl/mouse, was able to significantly increase gastric emptying (maximum effect: Formulation F1 41.1±0.6).

In contrast, composition F2 [which does not contain: i) a digestive enzyme, and ii) liquorice (*Glycyrrhiza glaba L*.) in the form of a liquid extract or dry extract] and composition F3 [which does not contain: (ii) liquorice *(Glycyrrhiza glaba L.)* in the form of a liquid extract or dry extract] administered at the same dose of 10 µl/mouse show percentages of gastric emptying comparable to the control group, which was not administered any composition.

It should be noted that, contrary to what one might have expected, formulation F3, which also contains the digestive enzyme actinidin (kiwi extract), when compared to formulation F2, has a decidedly smaller effect on gastric emptying than formulation F2.

### References

[1] Smits GJ, Lefebvre RA. Influence of aging on gastric emptying of liquids, small intestine transit, and fecal output in rats. Exp Gerontol. 1996; 31(5):589-96.

## Claims

1. A composition comprising a mixture which comprises:
(i) sodium alginate;
(ii) calcium carbonate;
(iii) sodium bicarbonate;
(iv) the digestive enzyme actinidin from *Actinidia chinensis Planch* (Kiwi) dry fruit extract, having a content of actinidin of 16000 CDU/g, and
(v) liquorice *(Glycyrrhiza glaba L.)* in the form of a dry root extract containing 3% glycyrrhizic acid.

2. The composition according to claim 1 wherein said mixture further comprises a mixture of plant extracts consisting of:
- *Opuntia ficus-indica Mill.* (prickly pear), preferably in the form of a dry cladode extract,
- *Olea europaea L.* (olive), preferably in the form of a dry leaf extract containing 3.7% polyphenols,
- *Mentha x piperita L.* (mint), preferably in the form of a dry leaf extract 1:4.

3. A composition according to any one of claims 1-2 for use as a medication.

4. A composition according to any one of claims 1-3 for oral use in the treatment of gastroesophageal reflux disease or disorders.

5. The composition for use according to claim 4, wherein the treatment of gastroesophageal reflux disease or disorders includes reducing gastric emptying times.

6. The composition for use according to claim 4 or 5, wherein the treatment of gastroesophageal reflux disease or disorders includes normalising and improving digestive function.

## Patentansprüche

1. Zusammensetzung, umfassend eine Mischung, welche umfasst:
(i) Natriumalginat;
(ii) Calciumcarbonat;
(iii) Natriumhydrogencarbonat;
(iv) das Verdauungsenzym Actinidin aus dem Trockenfruchtextrakt von *Actinidia chinensis Planch* (Kiwi) mit einem Actinidingehalt von 16 000 CDU/g, und
(v) Süßholz/Lakritze *(Glycyrrhiza glaba L.)* in Form eines Trockenwurzelextrakts mit 3 % Glycyrrhizinsäure.

2. Zusammensetzung nach Anspruch 1, wobei die Mischung ferner eine Mischung von Pflanzenextrakten umfasst, welche besteht aus:
- *Opuntia ficus-indica Mill.* (Kaktusfeige), vorzugsweise in Form eines Trockenkladodienextrakts,
- *Olea europaea L.* (Ölbaum), vorzugsweise in Form eines Trockenblattextrakts mit 3,7 % Polyphenol,
- *Mentha x piperita L.* (Pfefferminze), vorzugsweise in Form eines Trockenblattextrakts im Verhältnis 1:4.

3. Zusammensetzung nach einem der Ansprüche 1 bis 2, zur Verwendung als Medikament.

4. Zusammensetzung nach einem der Ansprüche 1 bis 3, für die orale Verwendung bei der Behandlung von gastroösophagealer Refluxkrankheit oder gastroösophagealen Störungen.

5. Zusammensetzung zur Verwendung nach Anspruch 4, wobei die Behandlung von gastroösophagealer Refluxkrankheit oder gastroösophagealen Störungen die Verringerung von Magenentleerungszeiten beinhaltet.

6. Zusammensetzung zur Verwendung nach Anspruch 4 oder 5, wobei die Behandlung von gastroösophagealer Refluxkrankheit oder von gastroösophagealen Störungen die Normalisierung und Verbesserung der Verdauungsfunktion beinhaltet.

## Revendications

1. Composition comprenant un mélange qui comprend :
(i) de l'alginate de sodium ;
(ii) du carbonate de calcium ;
(iii) du bicarbonate de sodium ;
(iv) l'enzyme digestive actinidine d'extrait sec de fruit d'*Actinidia chinensis Planch* (kiwi), ayant une teneur en actinidine de 16 000 CDU/g, et
(v) de la réglisse *(Glycyrrhiza glaba L.)* sous la forme d'un extrait sec de racine contenant 3 % d'acide glycyrrhizique.

2. Composition selon la revendication 1, dans laquelle ledit mélange comprend en outre un mélange d'extrait végétaux consistant en :
- *Opuntia ficus-indica Mill.* (figue de Barbarie), de préférence sous la forme d'un extrait sec de cladode,
- *Olea europaea L.* (olive), de préférence sous la forme d'un extrait sec de feuille contenant 3,7 % de polyphénols,
- *Mentha x piperita L.* (menthe), de préférence sous la forme d'un extrait sec de feuille au 1/4.

3. Composition selon l'une quelconque des revendications 1 et 2, pour une utilisation en tant que médicament.

4. Composition selon l'une quelconque des revendications 1 à 3, pour un usage oral dans le traitement de troubles ou d'une maladie de reflux gastro-œsophagien.

5. Composition pour une utilisation selon la revendication 4, dans laquelle le traitement de troubles ou d'une maladie de reflux gastro-œsophagien comprend la réduction des temps de vidage gastrique.

6. Composition pour une utilisation selon la revendication 4 ou 5, dans laquelle le traitement de troubles ou d'une maladie de reflux gastro-œsophagien comprend une normalisation et une amélioration de la fonction digestive.
